# EUROPEAN PATENT SPECIFICATION

(11) **EP 0 789 693 B1**
(45) Date of publication and mention of the grant of the patent: **07.01.1999**
(21) Application number: 95938402.5
(22) Date of filing: 31.10.1995
(51) Int. Cl.: C07D 301/10, B01J 23/66

(54) **PROCESS FOR PREPARING ETHYLENE OXIDE CATALYSTS**
VERFAHREN ZUR HERSTELLUNG VON ETHYLENOXULKATALYSATOREN
PROCEDE DE PREPARATION DE CATALYSEURS POUR LA PRODUCTION D'ACIDE D'ETHYLENE

(30) Priority: 01.11.1994 US 333016; 16.11.1994 US 340560
(43) Date of publication of application: 20.08.1997
(73) Proprietor: SHELL INTERNATIONALE RESEARCH MAATSCHAPPIJ B.V., 2596 HR Den Haag (NL)
(72) Inventor: EVANS, Wayne, Errol, Richmond, TX 77469 (US); KEMP, Richard, Alan, Stafford, TX 77477 (US)
(74) Representative: Zeestraten, Albertus Wilhelmus Joannes
(86) International application number: EP9504323
(87) International publication number: WO9613493

(56) References cited:
- EP-A- 0 266 015
- FR-A- 2 253 747
- US-A- 2 177 361
- US-A- 5 102 848

## Description

The invention relates to a process for the preparation of silver-containing catalysts suitable for the preparation of ethylene oxide and to the use of the catalyst for the preparation of ethylene oxide.

Catalysts for the production of ethylene oxide from ethylene and molecular oxygen are generally supported silver catalysts. Such catalysts are typically promoted. Small amounts of the alkali metals potassium, rubidium and cesium were noted as useful promoters in supported silver catalysts in U.S. Patent No. 3,962,136, issued June 8, 1976, and U.S. Patent No. 4,010,115, issued March 1, 1977. The use of other co-promoters, such as rhenium, or rhenium along with sulfur, molybdenum, tungsten and chromium is disclosed in U.S. Patent No. 4,766,105, issued August 23, 1988, and U.S. Patent No. 4,808,738, issued February 28, 1989. U.S. Patent No. 4,908,343, issued March 13, 1990, discloses a supported silver catalyst containing a mixture of a cesium salt and one or more alkali metal and alkaline earth metal salts.

It has now been found that catalysts pre-doped, pretreated or pre-impregnated with a promoting amount of one or more alkaline earth metals have higher initial activities than those obtained with catalysts which have not been thus pre-doped.

The invention therefore relates to a process for the preparation of a catalyst for the production of ethylene oxide from ethylene and molecular oxygen in the vapor phase which process comprises depositing a promoting amount of one or more alkaline earth metals on a porous, refractory support, at least partially drying the support, and thereafter depositing a catalytically effective amount of silver, a promoting amount of alkali metal, and optionally, a promoting amount of rhenium and/or a promoting amount of a rhenium co-promoter preferably selected from sulfur, molybdenum, tungsten, chromium, phosphorus, boron and mixtures thereof, on the support, and subsequently drying the support.

Preferably, the alkaline earth metal(s) is barium and/or magnesium.

Generally, in the vapor phase reaction of ethylene with oxygen to produce ethylene oxide, the ethylene is present in at least a double amount (on a molar basis) compared with oxygen, but frequently is often much higher. Therefore, the conversion is calculated according to the mole percentage of oxygen which has been consumed in the reaction to form ethylene oxide and any oxygenated by-products. The oxygen conversion is dependent on the reaction temperature, and the reaction temperature is a measure of the activity of the catalyst employed. The value T₄₀ indicates the temperature at 40 mole percent oxygen conversion in the reactor and is expressed in °C. This temperature for any given catalyst is higher when the conversion of oxygen is higher. Moreover, this temperature is strongly dependent on the employed catalyst and the reaction conditions. The selectivity (to ethylene oxide) indicates the molar amount of ethylene oxide in the reaction product compared with the total molar amount of ethylene converted. In this specification, the selectivity is indicated as S₄₀, which means the selectivity at 40 mole percent oxygen conversion, and the activity is indicated as T₄₀. The activity of the catalyst is important because lower temperatures can result in extended catalyst life, since undesired side reactions are minimized at lower temperatures. As used herein, "activity" is used to refer to the reaction temperature when measured at a given constant oxygen conversion level of 40% at a gas hourly space velocity of approximately 3300 and when measured after the ethylene oxide catalyst has been equilibrated under the initial set of reaction conditions.

The catalysts of the instant invention comprise a catalytically effective amount of silver, a promoting amount of alkali metal, optionally a promoting amount of rhenium, and optionally a promoting amount of a rhenium co-promoter selected from sulfur, chromium, molybdenum, tungsten, phosphorus, boron or mixtures thereof, supported on a porous, refractory support which has been pretreated with a promoting amount of alkaline earth metal(s).

In general, the catalysts of the present invention are prepared by impregnating porous refractory supports with compound(s), complex(es) and/or salt(s) of alkaline earht metal(s), in particular barium and/or magnesium dissolved in a suitable solvent sufficient to cause deposition on the support of from 10 to 8000, preferably from 15 to 1500 parts per million by weight of the total catalyst, of the alkaline earth metal(s). The porous refractory support is then at least partially dried and thereafter impregnated with silver ions or compound(s), complex(es) and/or salt(s) dissolved in a suitable solvent sufficient to cause deposition on the support of from 1 to 40, preferably from 1 to 25 percent by weight, basis the weight of the total catalyst, of silver. The impregnated support is subsequently separated from the solution and the deposited silver compound is reduced to metallic silver. Also deposited on the support either prior to, coincidentally with, or subsequent to the deposition of the silver will be suitable ions, or compound(s) and/or salt(s) of alkali metal dissolved in a suitable solvent. Optionally deposited on the support either prior to, coincidentally with, or subsequent to the deposition of the silver and/or alkali metal will be suitable rhenium ions or compound(s), complex(es) and/or salt(s) dissolved in an appropriate solvent, and/or suitable ions or salt(s), complex(es) and/or compound(s) of sulfur, molybdenum, tungsten, chromium, phosphorus and/or boron dissolved in an appropriate solvent. In the present invention, the alkaline earth metal compound(s), complex(es) and/or salt(s) dissolved in an appropriate solvent will be deposited on the carrier prior to the deposition of the silver, alkali metal, rhenium, if present, and rhenium co-promoter, if present.

The carrier or support employed in these catalysts in its broadest aspects can be any of the large number of conventional, porous refractory catalyst carriers or support materials which are considered relatively inert in the presence of ethylene oxidation feeds, products and reaction conditions. Such conventional materials are known to those skilled in the art and may be of natural or synthetic origin and preferably are of a macroporous structure, i.e., a structure having a surface area below 10 m²/g and preferably below 3 m²/g. Particularly suitable supports are those of aluminous composition, in particular those comprising alpha alumina. In the case of alpha alumina-containing supports, preference is given to those having a specific surface area as measured by the B.E.T. method of from 0.03 to 10, preferably from 0.05 to 5, more preferably from 0.1 to 3 m²/g, and a water pore volume as measured by conventional water absorption techniques of from 0.1 to 0.75 ml/g by volume. The B.E.T. method for determining specific surface area is described in detail in Brunauer, S., Emmet, P. Y. and Teller, E., J. Am. Chem. Soc., 60, 309-16 (1938).

The catalysts of the present invention are prepared by a technique in which an alkaline earth metal promoter in the form of soluble salts and/or compounds is deposited on the support which is then subjected to partial drying or to a thermal treatment sufficient to allow deposition of the barium salts and, while not wishing to be bound by any particular theory, presumed complexation with the anionic components on the surface of the catalyst. Thereafter, the alkali metal promoter(s), the rhenium promoter, if present, and the rhenium co-promoter, if present, in the form of soluble salts and/or compounds are deposited on the catalyst and/or support prior to, simultaneously with, or subsequent to the deposition of the silver and each other.

The preferred method is to deposit silver, alkali metal, rhenium and rhenium co-promoter simultaneously on the support, that is, in a single impregnation step.

The alkaline earth metal, however must be deposited on the support prior to all of the other catalyst components and the support must then be at least partially dried to a degree sufficient to fix the alkaline earth metal on the support before deposition of the other catalyst components. The pre-impregnation or pre-doping of the catalyst with a promoting amount of alkaline earth metal results in a catalyst having improved initial activity.

Generally, the carrier is contacted with an alkaline earth salt, compound or complex which has been dissolved in an aqueous or essentially aqueous solution, dried at a temperature in the range of from 200 °C to 1000 °C, and then contacted with a silver salt, a silver compound, or a silver complex which has been dissolved in an aqueous solution, so that the carrier is impregnated with said aqueous solution; thereafter the impregnated carrier is separated form the aqueous solution, e.g., by centrifugation or filtration and then dried. The thus obtained impregnated carrier is heated to reduce the silver to metallic silver. It is conveniently heated to a temperature in the range of from 50 °C to 600 °C, during a period sufficient to cause reduction of the silver salt, compound or complex to metallic silver and to form a layer of finely divided silver, which is bound to the surface of the carrier, both the exterior and pore surface. Air, or other oxidizing gas, reducing gas, an inert gas or mixtures thereof may be conducted over the carrier during this heating step.

Although alkaline earth metals exist in a pure metallic state, they are not suitable for use in that form. The alkaline earth metal is used as an ion or compound dissolved in a suitable solvent for impregnation purposes. The catalyst is pre-impregnated or pre-doped with a solution of alkaline earth promoter ions, salt(s) and/or compound(s) prior to the impregnation of the silver ions or salt(s), complex(es), and/or compound(s) and the other promoters, i.e., alkali metal, optionally rhenium, and optionally rhenium co-promoter.

The promoting amount of alkaline earth metal utilized will depend on several variables, such as, for example, the surface area and pore structure and surface chemical properties of the carrier used, the silver content of the catalyst and the particular ions used in conjunction with the alkali metal cation, rhenium, if present, or rhenium co-promoter, if present, and amounts of alkali metal, rhenium, if any and rhenium co-promoter, if any, present. The amount of alkaline earth metal promoter deposited upon the support or present on the catalyst generally lies between 10 and 8000, preferably between 15 and 1500, most preferably between 30 and 800 parts per million by weight of the total catalyst.

In a preferred embodiment, the alkaline earth compound is selected from the group consisting of barium and/or magnesium acetate, acetylacetonate, chloride, hydroxide, nitrate, oxide and mixtures thereof, with the nitrate, acetate, chloride and mixtures thereof being particularly preferred. Particularly preferred barium and magnesium promoters are the nitrate and the acetate.

The alkaline earth metal promoter or promoters are presumably present on the catalyst in the form of oxides or oxygen-bound species, or surface compounds or surface complexes rather than as metals. However, for purposes of convenience, the amount of alkaline earth deposited on the support or present on the catalyst is expressed as the element. Without intending to limit the scope of the invention, it is believed that the alkaline earth compounds are oxidic compounds. More particularly, it is believed that the alkaline earth compounds are probably in the form of mixed surface oxides or double surface oxides or complex surface oxides with the aluminum of the support and/or the silver of the catalyst, possibly in combination with species contained in or formed from the reaction mixture, such as, for example, chlorides or carbonates or residual species from the impregnating solution(s).

The carrier is impregnated with a solution of alkali metal promoter ions, salt(s) and/or compound(s) before, during or after impregnation of the silver ions or salt(s), complex(es), and/or compound(s) has taken place. An alkali metal promoter may even be deposited on the carrier after reduction to metallic silver has taken place.

The amount of alkali metal promoter deposited upon the support or present on the catalyst generally lies between 10 and 3000, preferably between 15 and 2000 and more preferably, between 20 and 1500, most preferably, between 50 and 1000 parts per million by weight of the total catalyst.

The alkali metal promoters are present on the catalysts in the form of cations or compounds of complexes rather than as the extremely active free alkali metals, although for convenience purposes in this specification and claims they are referred to as "alkali metal" or "alkali metal promoters". For purposes of convenience, the amount of alkali metal deposited on the support or present on the catalyst is expressed as the metal.

In a preferred embodiment, at least a major proportion (greater than 50%) of the alkali metals comprise the higher alkali metals, i.e. the alkali metals selected from the group consisting of potassium, rubidium, cesium and mixtures thereof.

A particularly preferred alkali metal promoter is cesium plus at least one additional alkali metal preferably selected from sodium, lithium and mixtures thereof, with lithium being preferred.

It should be understood that the amounts of alkali metal promoters on the catalysts are not necessarily the total amounts of these metals present in the catalyst.

These amounts do not include amounts of alkali metals which are locked into the support, for example, by calcining, or are not extractable in a suitable solvent such as water or lower alkanol or amine or mixtures thereof and do not provide a promoting effect. It is also understood that a source of the alkali metal promoter ions, salts and/or compounds used to promote the catalyst may be the carrier. That is, the carrier may contain extractable amounts of alkali metal that can be extracted with a suitable solvent, such as water or lower alkanol.

In one embodiment, the carrier is also impregnated with rhenium ions, salt(s), compound(s), and/or complex(es). This may be done at the same time that the alkali metal promoter is added, or before or later; or at the same time that the silver is added, or before or later; or at the same time that the rhenium co-promoter, if present, is added, or before or later. Preferably, rhenium, alkali metal, rhenium co-promoter, if present, and silver are in the same impregnating solution. When a rhenium promoter is utilized, the preferred amount of rhenium, calculated as the metal, deposited on or present on the carrier or catalyst ranges from 0.1 to 10, more preferably from 0.2 to 5 micromoles per gram of total catalyst, or, alternatively stated, from 19 to 1860, more preferably from 37 to 930 parts per million by weight of total catalyst.

Suitable rhenium compounds for use in the preparation of the instant catalysts are rhenium compounds that can be solubilized in an appropriate solvent. Preferably, the solvent is a water-containing solvent. More preferably, the solvent is the same solvent used to deposit the silver and the alkali metal promoter. Examples of suitable rhenium compounds include the rhenium salts such as rhenium halides, oxyhalides, rhenates, perrhenates, the oxides and the acids of rhenium. A preferred compound for use in the impregnation solution is the perrhenate, preferably ammonium perrhenate. However, the alkali metal perrhenates, alkaline earth metal perrhenates, silver perrhenates, other perrhenates and rhenium heptoxide can also be suitably utilized.

In a preferred embodiment of the instant invention, the rhenium present on the catalyst is present in a form that is extractable in a dilute aqueous base solution.

U.S. Patent No. 4,766,105, teaches that if a rhenium co-promoter selected from sulfur, molybdenum, tungsten and/or chromium is added to an alkali metal/rhenium doped supported silver catalyst, an improvement in initial selectivity is obtained. While suitable catalysts can be prepared in the absence of both rhenium and a rhenium co-promoter, it is preferable that if the catalyst contains rhenium, the catalyst also contains a rhenium co-promoter. When a co-promoter is utilized in the present invention, it is selected from the group consisting of (preferably a compound of) sulfur, molybdenum, tungsten, chromium, phosphorus, boron and mixtures thereof. In a presently preferred embodiment, the co-promoter is applied to the catalyst in the oxyanionic form.

Preferred are sulfates, molybdates, tungstates, chromates, phosphates and borates. The anions can be supplied with various counter-ions. Preferred are ammonium, alkali metal, mixed alkali metal and hydrogen (i.e. acid form).

The preferred amount of rhenium co-promoter compound present on or deposited on the support or catalyst ranges from 0.1 to 10, preferably from 0.2 to 5 micromoles, expressed as the element, per gram of total catalyst.

One method of preparing the silver containing catalyst can be found in U.S. Patent 3,702,259. Other methods for preparing the silver-containing catalysts which in addition contain higher alkali metal promoters can be found in U.S. Patent 4,010,115; U.S. Patent 4,356,312; U.S. Patent 3,962,136; and U.S. Patent 4,012,425.

Methods for preparing silver-containing catalysts containing higher alkali metal and rhenium promoters can be found in U.S. Patent No. 4,761,394, and methods for silver-containing catalysts containing higher alkali metal and rhenium promoters and a rhenium co-promoter selected from sulfur, molybdenum, tungsten and chromium can be found in U.S. Patent No. 4,766,105.

The preferred amount of alkali metal promoter deposited on or present on the surface of the carrier of catalyst generally lies between 10 and 3000, preferably between 15 and 2000 and more preferably between 20 and 1500 parts per million by weight of alkali metal calculated on the total carrier material. Amounts between 50 and 1000 parts per million are most preferable. Suitable compounds of alkali metals comprise lithium, sodium, potassium, rubidium, cesium or mixtures thereof in a promoting amount with the even more preferred promoters being rubidium and/or cesium plus an additional alkali metal selected from lithium, sodium and mixtures thereof.

The most preferred promoter is cesium plus lithium, preferably applied in an aqueous solution having cesium nitrate or cesium hydroxide dissolved therein.

In general terms, the impregnation process comprises impregnating the support with one or more solutions comprising alkaline earth metal, at least partially drying the impregnated support, impregnating the alkaline earth pre-doped support with one or more solutions comprising silver, alkali metal, and optionally, rhenium and rhenium co-promoter, and drying the support. As used in the instant specification and claims, the terminology "impregnating the support with one or more solutions comprising silver, alkali metal, rhenium and/or rhenium co-promoter", and similar or cognate terminology means that the support is impregnated in a single or multiple impregnation with one solution containing silver, alkali metal, rhenium and rhenium co-promoter in differing amounts; or in multiple impregnations with two or more solutions, wherein each solution contains at least one component selected from silver, alkali metal, rhenium and rhenium co-promoter, with the proviso that all of the components of silver, alkali metal, rhenium and rhenium co-promoter will individually be found in at least one of the solutions. For purposes of the pre-impregnation step, the concentration of the alkaline earth (expressed as the element) will range from 1 x 10⁻⁵ up to 1 g/l and preferably, from 5 x 10⁻⁴ to 0.1 g/l when a single pre-impregnation step is utilized. The concentration of the silver (expressed as the metal) in the silver-containing solution will range from 1 g/l up to the solubility limit when a single impregnation is utilized. The concentration of the alkali metal (expressed as the metal) will range from 1 x 10⁻³ up to 12 g/l and preferably, from 10 x 10⁻³ to 12 g/l when a single impregnation step is utilized. The concentration of the rhenium (expressed as the metal), if present, will range from 5 x 10⁻³ to 20 g/l and preferably from 50 x 10⁻³ to 20 g/l when a single impregnation step is utilized. The concentration of rhenium co-promoter (expressed as the element), if present, will range from 1 x 10⁻³ to 20 g/l and preferably from 10 x 10⁻³ to 20 g/l when a single impregnation step is utilized. Concentrations selected within the above noted ranges will depend upon the pore volume of the catalyst, the final amount desired in the final catalyst and whether the impregnation is single or multiple. Appropriate concentrations can be readily determined by routine experimentation.

The amount of silver deposited on the support or present on the support is to be a catalytically effective amount of silver, i.e., an amount that catalyzes the reaction of ethylene and oxygen to produce ethylene oxide. Preferably this amount will range from 1 to 40, more preferably from 1 to 25, and even more preferably from 5 to 20 percent by weight of the total catalyst.

The silver catalysts according to the present invention have been shown to have high initial activities for ethylene oxide production in the direct oxidation of ethylene with molecular oxygen to ethylene oxide. The conditions for carrying out such an oxidation reaction in the presence of the silver catalysts according to the present invention broadly comprise those already described in the prior art. This applies, for example, to suitable temperatures, pressures, residence times, diluent materials such as nitrogen, carbon dioxide, steam, argon, methane or other saturated hydrocarbons, to the presence of moderating agents to control the catalytic action, for example, 1-2-dichloroethane, vinyl chloride, ethyl chloride or chlorinated polyphenyl compounds, to the desirability of employing recycle operations or applying successive conversations in different reactors to increase the yields of ethylene oxide, and to any other special conditions which may be selected in processes for preparing ethylene oxide. Pressures in the range of from atmospheric to about 3500 KPa are generally employed. Higher pressures, however, are not excluded. Molecular oxygen employed as reactant can be obtained from conventional sources. The suitable oxygen charge may consist essentially or relatively pure oxygen, a concentrated oxygen stream comprising oxygen in major amount with lesser amounts of one or more diluents, such as nitrogen and argon, or another oxygen-containing stream, such as air. It is therefore evident that the use of the catalysts according to the present invention in ethylene oxide reactions is in no way limited to the use of specific conditions among those which are known to be effective. For purposes of illustration only, the following table shows the range of conditions that are often used in current commercial ethylene oxide reactor units.

**TABLE I**

| | |
|---|---|
| *GHSV | 1500-10,000 |
| Inlet Pressure | 1200-3000 kPa |
| Inlet Feed | |
| Ethylene | 1-40% |
| O₂ | 3-12% |
| Ethane | 0-3% |
| Argon and/or methane and/or nitrogen diluent | Balance |
| Chlorohydrocarbon Moderator | 0.3-50 ppmv total |
| Coolant temperature | 180-315 °C |
| Catalyst temperature | 180-325 °C |
| O₂ conversion level | 10-60% |
| EO Production (Work Rate) | 32-256 kg EO/m³ of catalyst/hr. |

| | |
|---|---|
| * Volume units of gas at standard temperature and pressure passing over one volume unit of packed catalyst per hour. | |

In a preferred application of the silver catalysts according to the present invention, ethylene oxide is produced when an oxygen-containing gas is contacted with ethylene in the presence of the present catalysts at a temperature in the range of from 180 °C to 330 °C and preferably 200 °C to 325 °C.

The invention will be illustrated by the following illustrative embodiments.

### Illustrative Embodiment

### Part A: Preparation of stock silver oxalate/ethylenediamine solution for use in catalyst preparation:

1) Dissolve 415 grams (g) of reagent-grade sodium hydroxide in 2340 millilitres (ml) deionized water. Adjust the temperature to 50 °C.
2) Dissolve 1699 g of "Spectropure" (high purity) silver nitrate in 2100 ml deionized water. Adjust the temperature to 50 °C.
3) Add sodium hydroxide solution slowly to silver nitrate solution with stirring while maintaining a temperature of 50 °C. Stir for 15 minutes after addition is complete, and then lower the temperature to 40 °C.
4) Insert clean filter wands and withdraw as much water as possible from the precipitate created in step (3) in order to remove sodium and nitrate ions. Measure the conductivity of the water removed and add back As much fresh deionized water as was removed by the filter wands. Stir for 15 minutes at 40 °C. Repeat this process until the conductivity of the water removed is less than 90 µmho/cm. Then add back 1500 ml deionized water.
5) Add 630 g of high-purity oxalic acid dihydrate in approximately 100 g increments. Keep the temperature at 40 °C and stir to mix thoroughly. Add the last portion of oxalic acid dihydrate slowly and monitor pH to ensure that pH does not drop below 7.8.
6) Remove as much water from the mixture as possible using clean filter wands in order to form a highly concentrated silver-containing slurry. Cool the silver oxalate slurry to 30 °C.
7) Add 699 g of 92 percent weight (%w) ethylenediamine (8% deionized water). Do not allow the temperature to exceed 30 °C during addition.

The above procedure yields a solution containing approximately 27-33%w silver.

### Part B: Catalyst pre-doping procedure:

For Catalyst A, in order to deposit 275 parts per million (ppm) (2 micromoles/gram) of barium ions, the following solution was made. 30 millilitres (ml) of monoethanolamine was diluted with 570 millilitres of deionized water to yield a total volume of 600 millilitres. Barium nitrate (0.6744 grams) was dissolved in the above solution. For catalyst A, 400 grams of Catalyst carrier I (99 wt% alpha alumina, B.E.T. Surface Area 0.48 m²/g, Water Pore Volume 0.465 ml/g) was then vacuum impregnated at 3.33-6.66 kPa for three minutes. At the end of this time, the vacuum was released and the excess solution is decanted from the carrier. The carrier was then dried by continuous shaking in a 3500 litre/hr air stream at 270 °C for seven minutes and four hours at 600 °C in a forced air oven.

For Catalyst B, the same procedure set forth for Catalyst A above was followed, except that the amount of barium nitrate was reduced by a factor of 4, i.e., 0.1686 grams.

For Catalyst C and D, no barium pre-doping of Catalyst carrier I was carried out.

For Catalyst E, in order to pre-deposit 4500 parts per million (ppm) of magnesium ions, the following solution was made. 5.74 grams of magnesium nitrate hetahydrate was dissolved in 60.0 ml deionized water. 120 grams of Catalyst carrier II (98.5 wt% alpha alumina, B.E.T. surface area 0.65 m²/g, Water Pore Volume 0.320 ml/g) was then vacuum impregnated at 3.33-6.66 kPa for three minutes. At the end of this time, the vacuum was released and the excess solution was decanted from the carrier. The carrier was then dried by continuous shaking in a 8500 litre/hr. air stream at 270 °C, three minutes at 120-150 °C, and four minutes at 250 °C.

For Catalyst F, no magnesium pre-doping of Catalyst carrier II was carried out.

For Catalyst G, in order to pre-deposit 48 parts per million (ppm) (2 micromoles/gram) of magnesium ions, the following solution was made. 30 millilitres (ml) of monoethanolamine was diluted with 570 millilitres of deionized water to yield a total volume of 600 millilitres. Magnesium acetate tetrahydrate (0.5535 grams) was dissolved in the above solution. 400 grams of Catalyst carrier I was then vacuum impregnated at 3.33-6.66 kPa for three minutes. At the end of this time, the vacuum was released and the excess solution was decanted from the carrier. The carrier was then dried by continuous shaking in a 8500 litre/hr air stream for seven minutes at 270 °C, and thereafter dried further in a forced air oven for four hours at 600 °C.

For Catalyst H, no magnesium pre-doping of Catalyst carrier I was carried out.

### Part C: Preparation of impregnated catalysts:

### Catalyst A

For preparing impregnated Catalyst A, a solution was prepared by adding cesium hydroxide (0.1458 grams) to 152 g of the above-prepared silver oxalate/ethylenediamine solution (specific gravity = 1.553 g/ml). 50 Grams of the resulting solution is used to prepare a catalyst with a target cesium level of 255 ppm.

The impregnation solution thus prepared was then used to impregnate a barium pre-doped carrier in the manner described below.

Approximately 30 g of the barium pre-doped carrier described above for Catalyst A were placed under 3.33 kPa for 3 minutes at room temperature. Approximately 50 g of the cesium-doped impregnating solution was then introduced to submerge the carrier, and the vacuum was maintained at 25 mm for an additional 3 minutes. At the end of this time, the vacuum was released, and excess impregnating solution removed from the carrier by centrifugation for 2 minutes at 500 rpm. The impregnated carrier was then cured by being continuously shaken in a 8500 litre/hr air stream at 250 °C for 5 minutes (over a cross-section area of approximately 19-32 cm²). The cured barium pre-doped catalyst (Catalyst A) was then ready for testing.

### Catalyst B

Catalyst B was prepared in the same manner as Catalyst A, except that a target cesium level of 270 ppm (0.1544 grams of cesium hydroxide) was used.

### Catalyst C

Catalyst C was prepared in the same manner as Catalyst A, except that the catalyst carrier was not pre-doped with barium.

### Catalyst D

Catalyst D was prepared in the same manner as Catalyst A, except that the catalyst carrier was not prep-doped with barium, but was coincidently doped with 275 parts per million (2 micrograms/gram) of barium ions.

The procedures set forth above for Catalysts A, B, C and D yielded catalysts on this carrier which contained approximately 13.5%w Ag with the following approximate dopant levels (expressed in parts per million by weight basis the weight of the total catalyst, i.e., ppmw) and which were approximately optimum in cesium for the given silver and rhenium, if present, and sulfur levels and support with regard to initial selectivity under the test conditions described below.

| | Barium, ppmw | Cesium, ppmw |
|---|---|---|
| Catalyst A | 275 | 255 |
| Catalyst B | 69 | 270 |
| Catalyst C | None | 250 |
| Catalyst D | 275* | 255 |

| | | |
|---|---|---|
| * coincidentally doped | | |

### Catalyst E

For preparing impregnated Catalyst E, into a 10 ml beaker was added 0.168 g of (NH₄)ReO₄ and approximately 2 g of ethylenediamine/H₂O (50/50 by weight), and the mixture was allowed to dissolve with stirring. 0.080 g of Li₂SO₄.H₂O was dissolved in 1 ml of water in a weighing dish, and then added to the perrhenate solution. 0.345 g of LiNO₃ was dissolved in 2 ml of water and added to the perrhenate solution. The perrhenate/lithium sulfate/lithium nitrate solution was allowed to stir, ensuring complete dissolution. This dopant solution was then added to 190 g of the above-prepared silver solution (specific gravity = 1.55 g/cc), and the resulting solution diluted with water to a total weight of 204 g. One-fourth of this solution was used to prepare a catalyst. 0.069 g of CsOH was added to a 51 g portion of the silver oxalate/dopant solution to prepare the final impregnation solution.

The final impregnation solution thus prepared was then used to impregnate a magnesium pre-doped carrier in the manner described below.

Approximately 30 g of the magnesium pre-doped carrier described above for Catalyst E were placed under 3.33 kPa for 3 minutes at room temperature. Approximately 50 g of doped impregnating solution was then introduced to submerge the carrier, and the vacuum maintained at 2.5 mm for an additional 3 minutes. At the end of this time, the vacuum was released, and excess impregnating solution removed from the carrier by centrifugation for 2 minutes at 500 rpm. Then the impregnated carrier was cured by being continuously shaken in a 8500 litre/hr air stream flowing across a cross-sectional area of approximately 19-32 cm² at 250-270 °C for 5-6 minutes. The cured magnesium pre-doped catalyst (Catalyst E) was then ready for testing.

### Catalyst F

Catalyst F was prepared in the same manner as Catalyst E, except that the catalyst carrier was not pre-doped with magnesium.

### Catalyst G

For preparing impregnated Catalyst G, a solution was prepared by adding cesium hydroxide (0.3375 grams) to 350 g of the above-prepared silver oxalate/ethylenediamine solution (specific gravity = 1.553 g/ml). 50 Grams of the resulting solution was used to prepare a catalyst with a target cesium level of 255 ppm.

The impregnation solution thus prepared was then used to impregnate a magnesium pre-doped carrier in the manner described below.

Approximately 30 g of the magnesium pre-doped carrier described above for Catalyst G were placed under 3.33 kPa for 3 minutes at room temperature. Approximately 50 g of the cesium-doped impregnating solution was then introduced to submerge the carrier, and the vacuum maintained at 3.33 kPa for an additional 3 minutes. At the end of this time, the vacuum was released, and excess impregnating solution removed from the carrier by centrifugation for 2 minutes at 500 rpm. The impregnated carrier was the cured by being continuously shaken in a 8500 litre/hr air stream at 250 °C for 5 minutes (over a cross-section area of approximately 19-32 cm²). The cured magnesium pre-doped catalyst (Catalyst G) was then ready for testing.

### Catalyst H

Catalyst H was prepared in the same manner as Catalyst G, except that the catalyst carrier was not pre-doped with magnesium.

The procedures set forth above for Catalysts E, F, G and H yielded catalysts on this carrier which contained approximately 13.5%w Ag with the following approximate dopant levels (expressed in parts per million by weight basis the weight of the total catalyst, i.e., ppmw) and which were approximately optimum in cesium for the given silver and rhenium, if present, and sulfur levels and support with regard to initial selectivity under the test conditions described below.

| | Magnesium, ppmw | Cs, ppmw | Re, ppmw | S, ppmw |
|---|---|---|---|---|
| Catalyst E | 4,000 | 600 | 280 | 48 |
| Catalyst F | None | 600 | 280 | 48 |
| Catalyst G | 48 | 255 | None | None |
| Catalyst H | None | 270 | None | None |

The actual silver content of the catalyst can be determined by any of a number of standard, published procedures. The actual level of rhenium on the catalysts prepared by the above process can be determined by extraction with 20 mM aqueous sodium hydroxide, followed by spectrophotometric determination of the rhenium in the extract. The actual level of barium on the catalyst can be determined by direct coupled plasma spectroscopy. The actual level of magnesium on the catalyst can be determined by standard atomic emission spectroscopy. The actual level of cesium on the catalyst can be determined by employing a stock cesium hydroxide solution, which has been labeled with a radioactive isotope of cesium, in catalyst preparation. The cesium content of the catalyst can then be determined by measuring the radioactivity of the catalyst. Alternatively, the cesium content of the catalyst can be determined by leaching the catalyst with boiling deionized water. In this extraction process cesium, as well as other alkali metals, is measured by extraction from the catalyst by boiling 10 grams of whole catalyst in 20 millilitres of water for 5 minutes, repeating the above two more times, combining the above extractions and determining the amount of alkali metal present by comparison to standard solutions of reference alkali metals using atomic absorption spectroscopy (using Perkin Elmer Model 1100 B or equivalent).

### Part D: Standard Microreactor Catalyst Test

### Conditions/Procedure:

3 to 5 grams of crushed catalyst (1.41-0.84 mm, i.e. 14-20 mesh) are loaded into a ¼ inch diameter stainless steel U-shaped tube. The U tube is immersed in a molten metal bath (heat medium) and the ends are connected to a gas flow system. The weight of the catalyst used and the inlet gas flow rate are adjusted to achieve a gas hourly space velocity of 3300 ml of gas per ml of catalyst per hour. The inlet gas pressure is 1550 kPa.

The gas mixture passed thorough the catalyst bed (in once-through operation) during the entire test run (including startup) consists of 30% ethylene, 8.5% oxygen, 5% carbon dioxide, 54.5% nitrogen, and 2.0 to 6.0 ppmv ethyl chloride.

The initial reactor (heat medium) temperature is 225 °C. After 1 hour at this initial temperature, the temperature is increased to 235 °C for 1 hour, and then adjusted to 245 °C for 1 hour. The temperature is then adjusted so as to achieve a constant oxygen conversion level of 40%. Performance data at this conversion level are usually obtained when the catalyst has been onstream for a total of at least 1-2 days. Due to slight differences in feed gas composition, gas flow rates, and the calibration of analytical instruments used to determine the feed and product gas compositions, the measured selectivity and activity of a given catalyst may vary slightly from one test run to the next. To allow meaningful comparison of the performance of catalysts tested at different times, all catalysts described in this illustrative embodiment were tested simultaneously with a reference catalyst. All performance data reported in this illustrative embodiment are corrected relative to the average initial performance of the reference catalyst, Catalyst C (S₄₀ = 81.0%; T₄₀ = 230 °C).

The results are presented below in Tables II and III. All selectivity values are expressed as % and all activity values are expressed as °C.

**TABLE II**

| Initial Performance of Catalysts A-D | | |
|---|---|---|
| (Data Obtained at 40% Conversion Conditions) | | |
| Catalyst | Initial Selectivity | Initial Activity |
| A | 81.0% | 224 °C |
| B | 81.5% | 224 °C |
| C | 81.0% | 230 °C |
| D | 81.0% | 230 °C |

As can be seen from Table II, catalysts prepared using a pre-doped barium carrier (Catalysts A and B) had higher initial activities than catalysts prepared without using a pre-doped barium carrier (Catalysts C and D), and were thus significantly advantaged. It can also be seen that the activity advantages of Catalysts A and B was obtained without sacrificing initial selectivity.

**TABLE III**

| Initial Performance of Catalysts E-H | | |
|---|---|---|
| (Data Obtained at 40% Conversion Conditions) | | |
| Catalyst | Initial Selectivity | Initial Activity |
| E | 89.6% | 253 °C |
| F | 89.3% | 258 °C |
| G | 81.4% | 222 °C |
| H | 81.0% | 230 °C |

As can be seen from Table III, catalysts which were prepared using a pre-doped magnesium carrier, both the rhenium-containing catalyst (Catalyst E) and the non-rhenium containing catalyst (Catalyst G), had higher initial activities than rhenium-containing and non-rhenium containing catalysts prepared without using a pre-doped magnesium carrier, Catalysts F and H respectively, and were thus significantly advantaged. It can also be seen that the activity advantages of Catalysts E and G were obtained without sacrificing initial selectivity.

## Claims

1. A process for preparing a catalyst for the vapor phase production of ethylene oxide from ethylene and oxygen which process comprises depositing a promoting amount of one or more alkaline earth metals on a porous, refractory support, at least partially drying the support, and thereafter depositing a catalytically effective amount of silver, a promoting amount of alkali metal and optionally a promoting amount of rhenium and a promoting amount of a rhenium co-promoter on said support, and thereafter drying the support.

2. The process of claim 1, wherein the amount of alkaline earth metal promoter is in the range of from 10 to 8000 parts per million, expressed as the element, by weight of the total catalyst, the amount of silver is in the range of from 1 to 40 percent by weight of the total catalyst, the amount of alkali metal promoter is in the range of from 10 to 1500 parts per million, expressed as the metal, by weight of the total catalyst, the amount of the optional rhenium is in the range of from 0.1 to 10 micromoles, expressed as the metal, per gram of total catalyst and the amount of the optional rhenium co-promoter is in the range of from 0.1 to 10 micromoles, expressed as the metal, per gram of total catalyst.

3. The process of claim 1 or 2, wherein the alkaline earth is barium and/or magnesium.

4. The process of claim 3, wherein the barium and/or magnesium is applied to the support as a solubilized compound.

5. The process of claim 4, wherein the barium and/or magnesium compound is selected from acetate, acetylacetonate, chloride, hydroxide, nitrate, oxide and mixtures thereof.

6. The process of claim 1, wherein the support comprises alpha alumina, having a surface area in the range of from 0.05 to 5 m²/g.

7. The process of any one of claims 1-6, wherein the alkali metal is selected from potassium, rubidium, cesium, and mixtures thereof.

8. The process of claim 7, wherein the alkali metal comprises cesium plus at least one additional alkali metal.

9. The process of claim 8, wherein the alkali metal is cesium plus lithium.

10. The process of any one of claims 1-9, wherein the optional rhenium co-promoter is selected from the group of sulfur, molybdenum, tungsten, chromium, phosphorus, boron and mixtures thereof.

## Patentansprüche

1. Verfahren zur Herstellung eines Katalysators für die Dampfphasenproduktion von Ethylenoxid aus Ethylen und Sauerstoff, welches Verfahren ein Ablagern einer Promotormenge eines oder mehrerer Erdalkalimetalle auf einem porösen, feuerfesten Träger, ein zumindest partielles Trocknen des Trägers und hierauf ein Ablagern einer katalytisch wirksamen Menge von Silber, einer Promotormenge von Alkalimetall und gegebenenfalls einer Promotormenge von Rhenium und einer Promotormenge eines Rhenium-Copromotors auf diesem Träger und anschließend ein Trocknen des Trägers umfaßt.

2. Verfahren nach Anspruch 1, worin die Menge an Erdalkalimetallpromotor im Bereich von 10 bis 8000 Teilen pro Million, ausgedrückt als das Element, bezogen auf das Gewicht des Gesamtkatalysators, liegt, die Silbermenge im Bereich von 1 bis 40 Gew.-% des Gesamtkatalysators liegt, die Menge an Alkalimetallpromotor im Bereich von 10 bis 1500 Teilen pro Million, ausgedrückt als das Metall, bezogen auf das Gewicht des Gesamtkatalysators, liegt, die Menge des fakultativen Rheniums im Bereich von 0,1 bis 10 µMol, ausgedrückt als Metall, je Gramm Gesamtkatalysator liegt und die Menge des fakultativen Rhenium-Copromotors im Bereich von 0,1 bis 10 µMol, ausgedrückt als Metall, je Gramm Gesamtkatalysator liegt.

3. Verfahren nach Anspruch 1 oder 2, worin das Erdalkalimetall Barium und/oder Magnesium ist.

4. Verfahren nach Anspruch 3, worin das Barium oder Magnesium als eine solubilisierte Verbindung auf den Träger aufgebracht wird.

5. Verfahren nach Anspruch 4, worin die Barium- und/oder Magnesiumverbindung unter Acetat, Acetylacetonat, Chlorid, Hydroxid, Nitrat, Oxid und Gemischen hievon ausgewählt wird.

6. Verfahren nach Anspruch 1, worin der Träger α-Aluminiumoxid mit einer Oberfläche im Bereich von 0,05 bis 5 m²/g ist.

7. Verfahren nach einem der Ansprüche 1-6, worin das Alkalimetall unter Kalium, Rubidium, Cäsium und Gemischen hievon ausgewählt wird.

8. Verfahren nach Anspruch 7, worin das Alkalimetall Cäsium plus wenigstens einem weiteren Alkalimetall umfaßt.

9. Verfahren nach Anspruch 8, worin das Alkalimetall Cäsium plus Lithium ist.

10. Verfahren nach einem der Ansprüche 1-9, worin der fakultative Rheniumcopromotor aus der Gruppe Schwefel, Molybdän, Wolfram, Chrom, Phosphor, Bor und Gemischen hievon ausgewählt wird.

## Revendications

1. Procédé de préparation d'un catalyseur en vue de la production en phase vapeur de l'oxyde d'éthylène à partir de l'éthylène et de l'oxygène, lequel procédé se caractérise en ce que l'on dépose une quantité promotrice d'un ou plusieurs métaux alcalino-terreux sur un support réfractaire et poreux, on sèche au moins partiellement le support et on dépose ensuite une quantité catalytiquement efficace d'argent, une quantité promotrice de métal alcalin et, éventuellement, une quantité promotrice de rhénium et une quantité promotrice d'un copromoteur du rhénium, sur le support précité et on sèche ensuite le support.

2. Procédé suivant la revendication 1, caractérisé en ce que la quantité de promoteur à base de métal alcalino-terreux varie de 10 à 8000 parties par million, exprimées sous forme de l'élément, en poids du catalyseur total, la quantité d'argent varie de 1 à 40% en poids du catalyseur total, la quantité du promoteur à base de métal alcalin varie de 10 à 1500 parties par million, exprimées sous forme du métal, en poids du catalyseur total, la quantité du rhénium facultatif varie de 0,1 à 10 micromoles, exprimées sous forme du métal, par gramme de catalyseur total et la quantité du copromoteur du rhénium facultatif varie de 0,1 à 10 micromoles, exprimées sous forme du métal, par gramme de catalyseur total.

3. Procédé suivant la revendication 1 ou 2, caractérisé en ce que le métal alcalino-terreux est le baryum et/ou le magnésium.

4. Procédé suivant la revendication 3, caractérisé en ce que l'on applique le baryum et/ou le magnésium sur le support sous la forme d'un composé solubilisé.

5. Procédé suivant la revendication 4, caractérisé en ce que le composé du baryum et/ou du magnésium est choisi parmi les acétates, les acétylacétonates, chlorures, hydroxydes, nitrates, oxydes et leurs mélanges.

6. Procédé suivant la revendication 1, caractérisé en ce que le support comprend de l'alpha-alumine, possédant une surface spécifique qui varie de 0,05 à 5 m²/g.

7. Procédé suivant l'une quelconque des revendications 1 à 6, caractérisé en ce que l'on choisit le métal alcalin parmi le potassium, le rubidium, le césium et leurs mélanges.

8. Procédé suivant la revendication 7, caractérisé en ce que le métal alcalin comprend du césium plus ou moins un métal alcalin supplémentaire.

9. Procédé suivant la revendication 8, caractérisé en ce que le métal alcalin est le césium plus le lithium.

10. Procédé suivant l'une quelconque des revendications 1 à 9, caractérisé en ce que le copromoteur du rhénium facultatif est choisi dans le groupe formé par le soufre, le molybdène, le tunsgtène, le chrome, le phosphore, le bore et leurs mélanges.
